# EUROPEAN PATENT APPLICATION

(11) **EP 1 163 878 A1**
(43) Date of publication of application: **19.12.2001**
(21) Application number: 00112681.2
(22) Date of filing: 15.06.2000
(51) Int. Cl.: A61B 5/113, A61B 5/00

(54) **Device for body activity detection and processing**

(71) Applicant: THE PROCTER & GAMBLE COMPANY, Cincinnati, Ohio 45202 (US)
(72) Inventor: Barron, Bradford S., 1780 Wemmel (BE); Boswell, Emily Charlotte, Isleworth Middlesex TW7 7PP (GB); Dauger-Strauss, Corrine, 1040 Brussels (BE); Deflander, Joseph Fernand, 2990 Wespelaar (BE); Macgilp, Neil Archibald, Bankfield Surrey, GU5 0BX (GB); Van den Wouwen, Chris, 2100 Deurne (BE)
(74) Representative: Hirsch, Uwe Thomas

(57) **Abstract**

A device for monitoring body activity and arranged for stand-alone attachment to a body in use. The device comprises: an actimetry sensor (11) for measuring body activity, and storage means (12) for receiving data from the actimetry sensor and storing it. Means analyses the stored data to provide advisory information and a display (4) displays the advisory information to a user.

## Description

### Field of the invention

This invention relates to the detection of body activity, such as sleep patterns, and the analysis of data related to such functions for provision to a user.

### Background

In recent years there has been much study of body functions, such as sleep activity, and associated analysis of the relevance of such functions to the general health of the body and the body's need for appropriate body functions (such as sleep patterns) to occur on a regular basis for adequate periods of time. As part of this research numerous devices have been proposed to assist in such measurement and analysis.

For example, WO-A-9714354 discloses a device and corresponding method which collects data for analysing sleep disturbances so that such data can be interpreted by a specialist at a future date.

However, this type of device requires operation by a highly skilled user and provides analysis which is difficult to interpret by anybody other than a specialist, as well as being expensive and sometimes unreliable. Furthermore, it is unable to provide a detailed history over an extended time period for an individual.

Other systems are uncomfortable, cannot be worn for extended periods and/or cannot be worn without restricting body movement.

### Summary of the invention

According to the present invention there is provided a device for monitoring body activity and arranged for stand-alone attachment to a body in use, the device comprising:
an actimetry sensor for measuring body activity;
storage means for receiving data from the actimetry sensor and storing it;
means for analysing the stored data to provide advisory information; and
means for displaying the advisory information to a user.

The actimetry sensor may be an accelerometer such as a piezoelectric accelerometer, or may be a simple motion sensor on tilt switch, for example.

The body activity being monitored may be sleep, and/or waking activity.

The storage means may store data from the actimetry sensor together with temporal information. In such a case, the means for analysing the stored data provides processing based upon both body activity information and temporaled information to provide advisory information to the user.

The advisory information provided to the user may include an indication of the quality of the activity, such as the quality of the sleep, whether or not the duration of the activity is sufficient, an indication as to whether the total amount of the activity over an extended period is acceptable, as well other data related to other long term body activity, for example. The device can be configured to detect activity during the day. The body activity that is measured can, as well as being actual time slept, be the number of awakenings, an indication as to how the intermittent sleep was, time taken before sleep, the number of and length of sleep interruption, sleep proficiency, the number minutes immobile/moving, etc. A selection or all of this information can be provided to identify the least and most active times during the day.

The device may include an input (such as buttons) for receiving input data from a user, such as desired time to go to sleep, the need to awake early for a particular event, as well as possible information relating to the age of the user, their sex, as well as optionally additional information such as what they perceive their energy level to be.

The device may have one or more additional sensors to also measure body pulse rate variability, blood pressure variability or other body activities such as eyelid movement or respiration. In this case, sleep phases such as REM, slow light sleep, slow deep sleep, or paradoxical sleep may be monitored.

The device may be configured in the style of a wrist watch, and may be arranged to provide additional information to a user, such as time and date information. The device may have an alarm.

The means for displaying the advisory information may be a liquid crystal display or a plasma display, for example.

### Brief description of the figures

One example of the present invention will now be described with reference to the accompanying drawings, in which:
Figure 1 is a schematic perspective view of a device according to the present invention;
Figure 2 is a schematic diagram showing some of the functionality of the device of Figure 1;
Figure 3 is a diagram showing two possible displays from the device of Figure 1; and
Figure 4 is a schematic diagram of the internal components of the device of Figure 1.

### Detailed description of on example

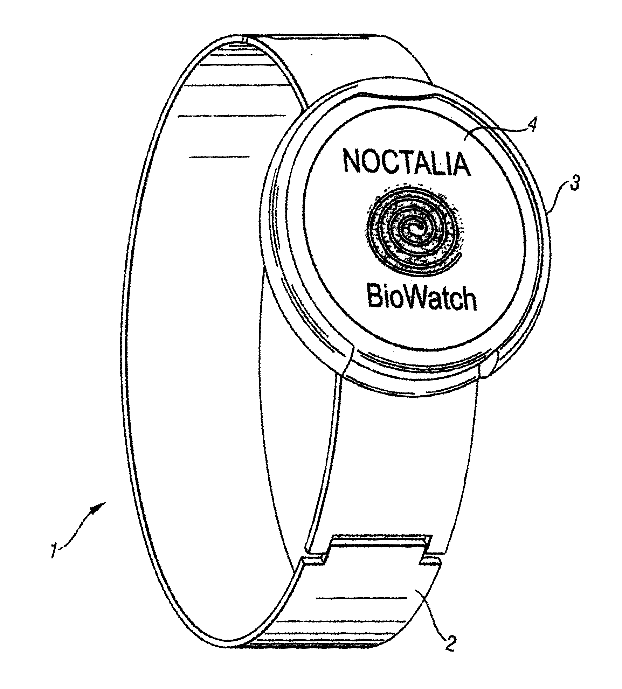

Referring to Figure 1, a device 1 according to the present invention is, in this example, configured as a wrist watch-style device (although could have a different configuration), with a strap 2 and a component-containing housing 3. On the outer surface of the housing 3 is a display 4, which in this case is a liquid crystal display.

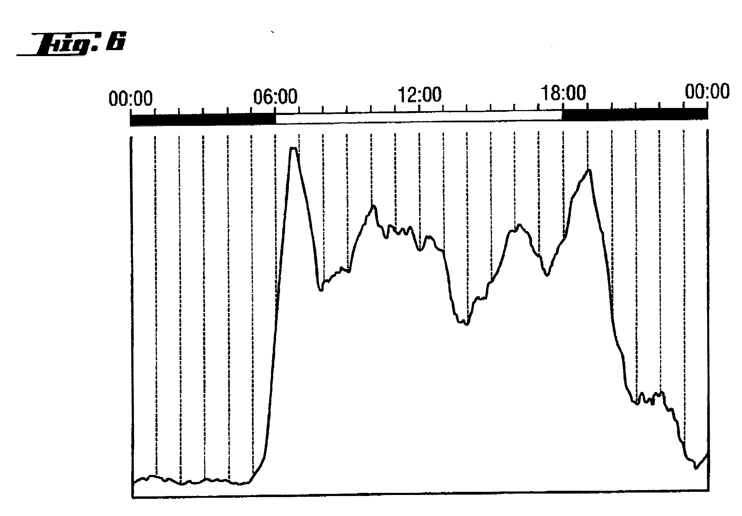

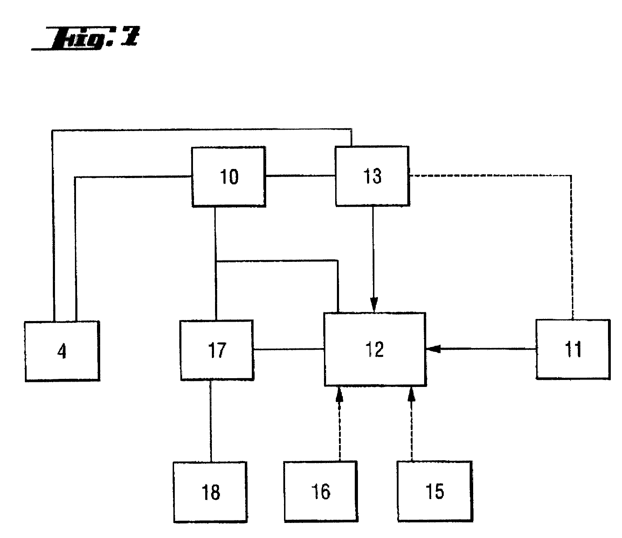

Referring to Figure 4, a device 1 of Figure 1 has a number of internal components. The device 1 is powered by a battery 10 (or another form of power supply) which supplies power to the other components of the device 1. An actimetry sensor 11 detects motion in the device 1 and hence motion in the body to which the device 1 is attached in use. The data from the actimetry sensor 11 is passed to a memory 12. A clock 13 also provides temporal data to the memory 12 and to the actimetry sensor 11 if necessary, as well as optionally to a display 4. In addition to the actimetry sensor 11 the device 1 may further comprise additional sensors 15, 16, which may detect blood pressure, pulse rate variability etc.

Data from these additional optional sensors 15, 16 may also be forwarded to the memory 12. Data from the memory 12 can be requested from analysing means 17, either on a regulated intermittent, continuous, or on a user-requested basis.

A wide variety of different forms of analysis may be performed by the analysing means 17.

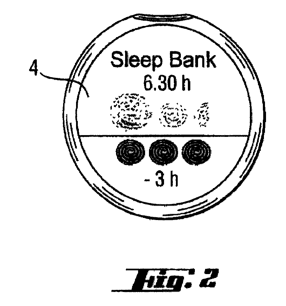

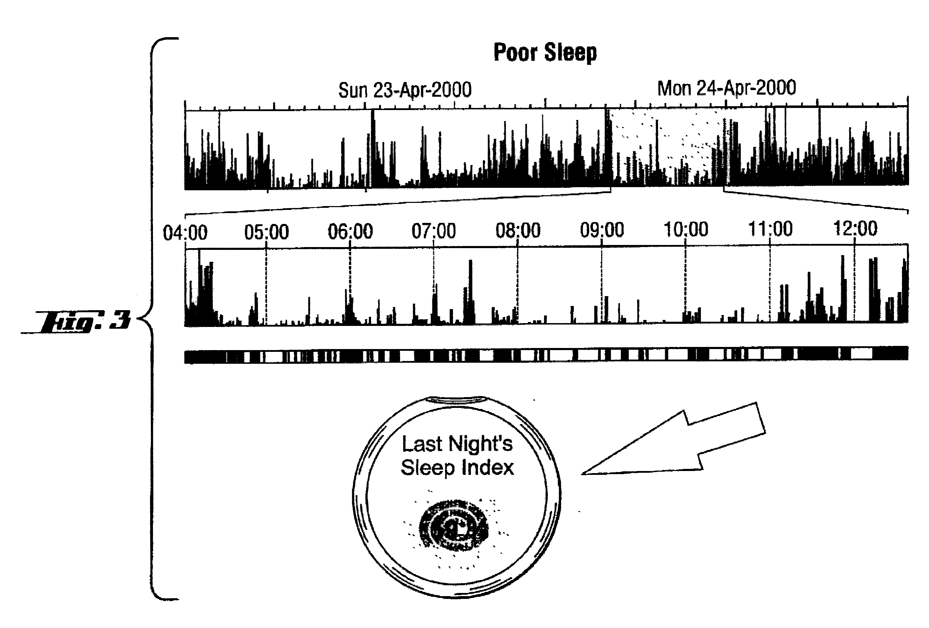

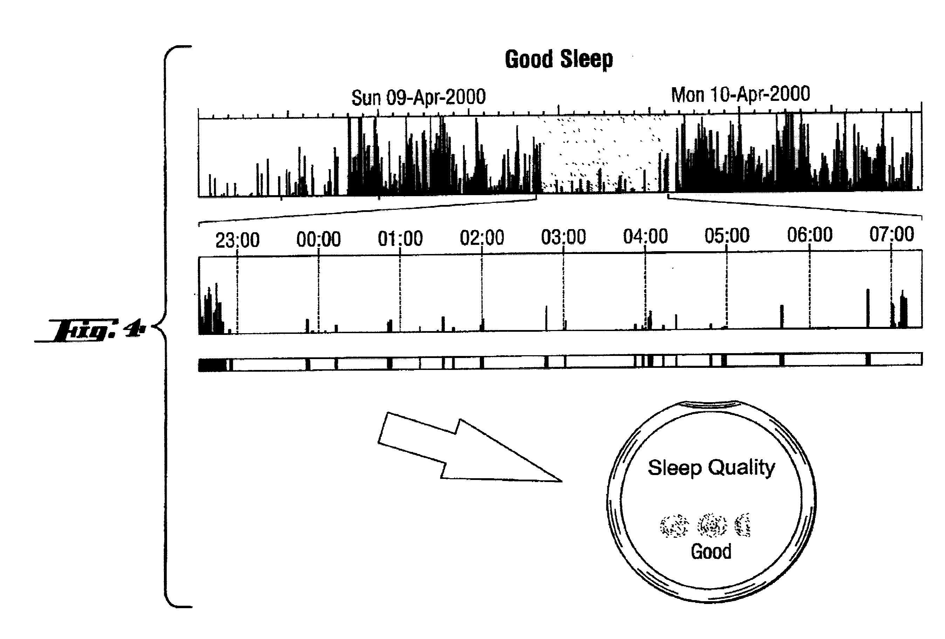

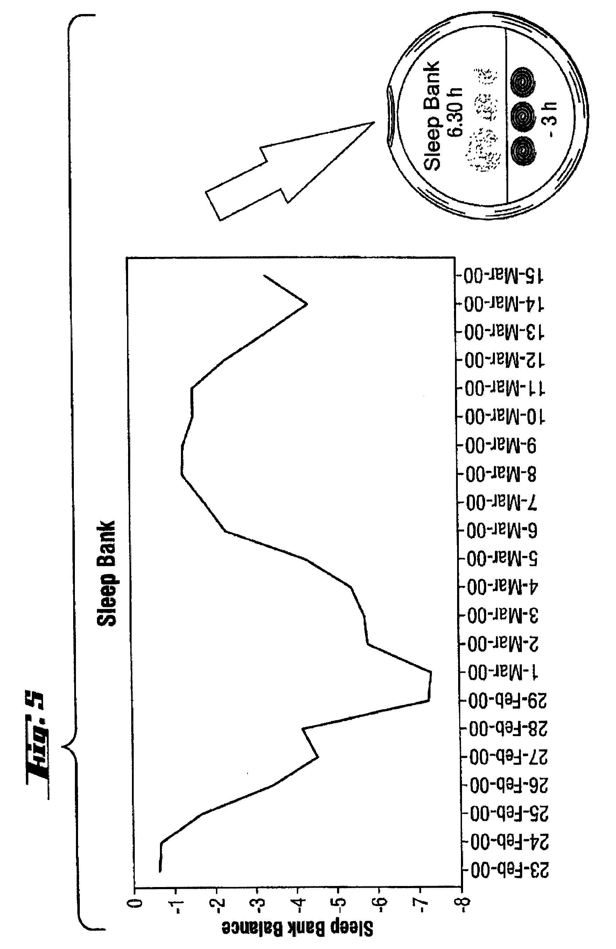

Examples of the types of analysis that may be performed will now be described with reference to Figures 2 and 3.

The actimetry sensor 11 may provide information in relation to sleep duration and the type of sleep to the analysing means 17. By type of sleep this may include the duration of sleep, number of interruptions, motion during sleep, for example. This information can be analysed by the analysing means 17 to provide information to the display form in terms simply of the total number of hours of effective sleep obtained, although it may provide additional information in relation to the quality of the sleep and the expected value of that sleep in terms of an "energy bank". By using data stored in the memory 12 over a number of days, weeks or months, the analysing means 17 may also provide information indicative of accumulated sleep deficit or sleep excess. As mentioned above, the data can be provided to a user as and when requested, and is arranged to be provided in a very simple format so that it does not need complex interpretation.

The analysing means 17 may employ a sleep scale such as the Stanford sleep scale in order to score the monitored sleep and receive relevant information from the user. The scale defines different levels of sleepiness as follows:
1 - feeling active, vital, alert, wide awake.
2 - functioning at a high level, not at peak.
3 - relaxed, not full alertness, responsive.
4 - a little soggy, not at peak, let down.
5 - tired, losing interest, slowed down.
6 - drowsy, prefer to be lying down.
7 - almost in a reverie, hard to stay awake.

This scale can be shown to a user so that the user can input an indication of how tired they consider themselves to be.

For example, the user could be prompted to input an indication as to how they feel when they wake up, with an indication as to the reasons for their feelings being provided by the analysing means 17 from the data collected.

In another example, such an input could be employed during the initial weeks of employing the device to help the device determined whether or not the user is sleeping for the right amount of time to them. For example, on the first day of wearing the device the device may prompt the user to indicate how much sleep they consider they need. It could then provide information as to the average sleep requirement for someone of their age and sex. However, as the requirements vary from user to user, the device can then monitor sleep over a given period and prompt the user for feedback, not only at that time but also during the day in order to form a sleep diary in the memory of the device.

The device may then be configured to adapt the indications that it gives the user based upon the feedback and wake the user at the appropriate time, and then employing a sleep bank once the user's particular requirements have been determined.

The device 1 may have an alarm 18, which can be used simply to wake the user, in the manner of a normal wrist watch alarm, although it may be activated by the analysing means 17, when it is detected that an appropriate type of sleep is occurring to ensure gentle waking of the user or waking at a time such that they have less sleep inertia.

If additional sensors 15, 16 are provided then additional analysis can be performed dependent upon the type of sensor.

If the sensors detect parameters external to the body, such as light, location, sound, air temperature, humidity, barometric pressure, then this information may be compared with information relating to body activity in order to adjust their information. If the sensors determine additional body activity, and detect one or more muscle tonus, skin temperature, galvanic skin response, etc then additional analysis of the quality of the sleep may be provided. As a further example, if a blood pressure sensor is employed then additional indications related to general levels of health and activity not specifically related to sleep alone can be provided by the analysing means. If a pulse rate variability detector is employed then this can assist in determining the type of sleep detected and the quality of that sleep, and can provide further information in relation to whether an acceptable level of aerobic exercise has been performed within the allotted time period, whether it be a day, a week or a month, for example.

If the device 1 provides some form of "sleep bank" indication over a period of time, then the sleep bank may calculate the information to be provided to the user by including a formula such as:
sleep bank (i) = sleep bank (i-1) + (sleep (i) - need) where sleep bank is accumulative of sleep balance on day i, sleep is sleep achieved on the night before day i and need is sleep need (which can change dependent upon other measured parameters, or upon stored data, or can be set manually).

## Claims

1. A device for monitoring body activity and arranged for stand-alone attachment to a body in use, the device comprising:
an actimetry sensor for measuring body activity;
storage means for receiving data from the actimetry sensor and storing it;
means for analysing the stored data to provide advisory information; and
means for displaying the advisory information to a user.

2. The device of claim 1, wherein the actimetry sensor is an accelerometer such as a piezoelectric accelerometer.

3. The device of claim 1 or 2, wherein the body activity being monitored is sleep.

4. The device of any preceding claim, wherein the storage means store data from the actimetry sensor together with temporal information.

5. The device of claim 5, wherein the means for analysing the stored data provides processing based upon both body activity information and temporaled information.

6. The device of any preceding claim, wherein the advisory information provided to the user includes an indication of the quality of the activity, including at least one of the quality of the sleep, whether or not the duration of the activity is sufficient, an indication as to whether the totalled amount of the activity over an extended period is acceptable, or other data related to other long term body activity.

7. The device of any preceding claim, further comprising at least one additional sensor measuring at least one of body pulse rate variability, and/or blood pressure.

8. The device of claim 7, wherein sleep phases of REM, slow light sleep, slow deep sleep, or paradoxical sleep are monitored.

9. The device of any preceding claim, wherein the device may be configured in the style of a wrist watch.

10. The device of claim 9, wherein the device has an alarm.

11. The device of any preceding claim, wherein the means for displaying the advisory information is a liquid crystal display.

12. The device of any preceding claim, further comprising a sensor for detecting data relating to the environment in which the body is placed.
